# EUROPEAN PATENT SPECIFICATION

(11) **EP 2 437 763 B1**
(45) Date of publication and mention of the grant of the patent: **21.10.2015**
(21) Application number: 10731818.0
(22) Date of filing: 28.05.2010
(51) Int. Cl.: A61K 36/48, A61P 3/10

(54) **A NOVEL ANTIDIABETIC FUROSTANOLIC SAPONIN RICH (FSR) FRACTION FROM FENUGREEK SEEDS**
NEUE ANTIDIABETISCHE FUROSTANOL-SAPONIN-REICHE (FSR) FRAKTION AUS FENCHELSAMEN
NOUVELLE FRACTION RICHE EN SAPONINE FUROSTANOLIQUE (FSR) ANTIDIABÉTIQUE EXTRAITE DE GRAINES DE FENUGREC

(30) Priority: 01.06.2009 IN DE11142009
(43) Date of publication of application: 11.04.2012
(73) Proprietor: Goel, Kumar Pawan, Panchkula 134109 (IN)
(72) Inventor: Goel, Kumar Pawan, Panchkula 134109 (IN)
(74) Representative: Grosset-Fournier, Chantal Catherine
(86) International application number: PCT/IN2010/000359
(87) International publication number: WO 2010/140165

(56) References cited:
- WO-A1-2009/153800
- SHARMA R D ET AL: "EFFECT OF FENUGREEK SEEDS ON BLOOD GLUCOSE AND SERUM LIPIDS IN TYPE I DIABETES", EUROPEAN JOURNAL OF CLINICAL NUTRITION, XX, XX, vol. 44, no. 4, 1 January 1990 (1990-01-01), pages 301-306, XP009047500, & SHARMA R D: "EFFECT OF FENUGREEK SEEDS AND LEAVES ON BLOOD GLUCOSE AND SERUM INSULIN RESPONSES IN HUMAN SUBJECTS", NUTRITION RESEARCH, ELSEVIER INC, XX, vol. 6, 1 January 1986 (1986-01-01), pages 1353-1364, XP002917085, ISSN: 0271-5317, DOI: DOI:10.1016/S0271-5317(86)80020-3
- SAUVAIRE Y ET AL: "STEROID SAPONINS FROM FENUGREEK AND SOME OF THEIR BIOLOGICAL PROPERTIES", ADVANCES IN EXPERIMENTAL MEDICINE AND BIOLOGY, SPRINGER, US, vol. 405, 1 January 1996 (1996-01-01), pages 37-46, XP009072535, ISSN: 0065-2598
- PETIT P R ET AL: "Steroid saponins from fenugreek seeds: Extraction, purification, and pharmacological investigation on feeding behavior and plasma cholesterol", STEROIDS, ELSEVIER SCIENCE PUBLISHERS, NEW YORK, NY, US, vol. 60, no. 10, 1 October 1995 (1995-10-01), pages 674-680, XP004026473, ISSN: 0039-128X, DOI: DOI:10.1016/0039-128X(95)00090-D
- MURAKAMI T ET AL: "MEDICINAL FOODSTUFFS. XVII. FENUGREEK SEED. (3): STRUCTURE OF NEW FUROSTANOL-TYPE STEROID SAPONINS, TRIGONEOSIDES XA, XB, XIB, XIIA, XIIB, AND XIIIA, FROM THE SEEDS OF EGYPTIAN TRIGONELLA FOENUM-GRAECUM L", CHEMICAL AND PHARMACEUTICAL BULLETIN, PHARMACEUTICAL SOCIETY OF JAPAN, TOKYO, JP, vol. 48, no. 7, 1 July 2000 (2000-07-01), pages 994-1000, XP001180963, ISSN: 0009-2363

## Description

### FIELD OF THE INVENTION

The field of invention pertains to herbal products having therapeutic applications. More specifically it pertains to a novel, anti-diabetic, chemically defined, furostanolic-saponin-rich (FSR) fraction (>70% including approximately 30% protodioscin as the major fraction) isolated from fenugreek seeds. Due to high bioactivity, the fraction can easily be formulated into a modern, pharmaceutically acceptable form e.g. capsule, tablet, syrup etc. for therapeutic use in Type-1 and Type-2 diabetes.

### BACKGROUND OF THE INVENTION:

### Diabetes

**Definition:** Diabetes refers to a group of diseases that lead to high blood glucose levels due to defects in either insulin secretion or insulin action. It is a syndrome of disordered metabolism, usually due to a combination of hereditary and environmental causes, resulting in abnormally high blood sugar levels (hyperglycemia). Blood glucose levels are controlled by a complex interaction of multiple chemicals and hormones in the body, including the hormone insulin made in the beta cells of the pancreas. Diabetes is a disease with lot of complications, showing a high mortality worldwide in line with other diseases such as cancer and cardiovascular disorder. The mortality of diabetes has persisted and there are many reports that the diabetic patients have a high risk of having some related complications in eye, kidney and heart.

**Causes:** Diabetes develops due to a diminished production of insulin (in type 1) or resistance to its effects (in type 2 and gestational). Both lead to hyperglycemia, which largely causes the acute signs of diabetes: excessive urine production, resulting compensatory thirst and increased fluid intake, blurred vision, unexplained weight loss, lethargy, and changes in energy metabolism.

**Classification:** It can be classified into 3 types as follows:
**1) Diabetes mellitus type 1 (insulin dependant)** : Type 1 diabetes mellitus is characterized by loss of the insulin-producing beta cells of the islets of Langerhans in the pancreas, leading to a deficiency of insulin. This type of diabetes can be further classified as immune-mediated or idiopathic (unknown reasons). The majority of type 1 diabetes is of the immune-mediated variety, where beta cell loss is a T-cell mediated autoimmune attack. Idiopathic factors include viruses and some toxic materials that may affect the immune source such as surface antigen of beta cell and insulin secreting cell in the pancreas.
**2) Diabetes mellitus type 2 (non- insulin dependant):** It is characterized by insulin resistance or reduced insulin sensitivity, combined with relatively reduced insulin secretion which in some cases becomes absolute. The defective responsiveness of body tissues to insulin almost certainly involves the insulin receptor in cell membranes. However, the specific defects are not known. Non insulin dependant diabetes is usually of gradual onset and occurs in people over 40 year old. Type 2 diabetes is the most common type. Unlike insulin-dependant diabetes, type 2 diabetes is called "adult diabetes" and its etiology (study of causes) is unknown though environmental factors responsible for diabetes includes high calorific food intake triggered by rapid economical growth in recent years, insufficient exercise, obesity, stress and drug over dose.
**3) Gestational diabetes:** Gestational diabetes mellitus (GDM) resembles type 2 diabetes in several respects, involving a combination of relatively inadequate insulin secretion and responsiveness. It occurs in about 2%-5% of all pregnancies and may improve or disappear after delivery. Gestational diabetes is fully treatable but requires careful medical supervision throughout the pregnancy. About 20%-50% of affected women develop type 2 diabetes later in life.
**4) Diabetes insipidus (DI):** It is another condition characterized by excessive thirst and excretion of large amounts of severely diluted urine, with reduction of fluid intake having no effect on the latter. It is not due to diminished insulin production, but is rather due to failure of pituitary gland to secrete anti-diuretic hormone (ADH).

### Pharmaceutical compositions for diabetes treatment

These can be classified into two broad categories- synthetic and natural (herbal). Synthetic pharmaceutical compositions include chemical compounds which have been synthesized whereas natural (herbal) pharmaceutical compositions are those which are extracted from plant materials. They may or may not contain additional non-plant ingredients viz. animal extracts/parts, minerals etc.

**Synthetic compositions:** There are several pharmaceutical compositions available in the market containing various types of chemicals and preservatives. The example of oral glucose lowering agents for the treatment of diabetes includes sulfonylureas, biguanides and acarbose. The second generation sulfonylureas have been frequently used, since their duration of action is short with a potent glucose lowering effect in non-obese diabetic patients, while biguanides and acarbose are indicated for obese diabetic patients exceeding the normal body weight. The marked difference among these anti-diabetic agents is that biguanides and acarbose have less incidence of hypoglycemia than sulfonylureas.

**Natural compositions (herbal):** Since ancient times, various herbs are being used to treat diabetes. Recent scientific investigations have confirmed the efficacy of many of these preparations, some of which are remarkably effective. Generally, herbal supplements are safe when taken as recommended, less expensive and sometimes a more effective alternative to synthetic drugs. However, the major disadvantages of herbal compositions/supplements are:
1. **Lack of standardization:** Most of the natural compositions are powdered mixtures of whole herbs. There is variation of the active components in the herbs due to seasonal and environmental conditions.
2. **Poor bioactivity:** Whole herbs and herbal mixtures suffer from the disadvantage of poor bioactivity necessitating consumption in large amounts, which is expensive and practically cumbersome for a patient e.g. a patient during travel may have to carry a large and heavy jar containing the mixture.
3. **Difficulty in converting such mixtures into modern, pharmaceutically acceptable forms:** Owing to poor bioactivity, large quantities of the herbs are required to be consumed by the patients. This makes formulation of such herbal mixtures into modern pharmaceutically acceptable forms e.g. capsules, tablets etc. very difficult and in some cases impossible. Additionally, analytical methods of quality control are difficult to develop owing to immense heterogeneity of the compounds in the mixture.
4. **Analytical challenges:** Standardization of whole herbal mixtures poses immense analytical challenges owing to large number of compounds in the mixture.
5. **Scarcity of raw materials:** Compositions based on raw materials derived from plants face the challenge of scarcity of raw materials. This may be due to seasonal nature of the source or even limited availability in the natural environment e.g. if tree parts are being used, not many trees can be 'sacrificed' for the same. The problem becomes acute when the part used is 'root' necessitating 'elimination' of the tree.

Some of the common herbs whose anti-diabetic activity is well-documented are:
1. Pterocarpus marsupium (Indian Kino, Malabar Kino, Pitasara, Venga)
2. Bitter Melon (Momordica charantia)
3. Gymnema Sylvestre (Gurmar, Meshasringi, Cherukurinja)
4. Onion and Garlic (Allium cepa and Allium sativum)
5. ***Fenugreek (Trigonella foenum-graecum)***
6. Blueberry leaves (Vaccinium myrtillus)
7. Asian Ginseng.
8. Stevia
9. Bilberry

The present invention pertains to a novel fraction derived from fenugreek seeds. The fraction comprises >70% furostanolic saponins including approximately 30% protodioscin as the major fraction. It shows powerful anti-diabetic activity and is highly bioactive, enabling convenient and easy formulation into modern pharmaceutically acceptable forms e.g. capsules, tablets, syrups etc. Also, it is chemically defined i.e. the components in the mixture and their quantities are well-defined due to which it can be subjected to modern methods of quality control and standardization. Non-toxic nature even at high concentrations makes it an attractive 'natural entity' in diabetes management and treatment.

Anti-diabetic property of Fenugreek as disclosed in the prior art is discussed below. Fenugreek belongs to the member of bean family and has been used for centuries as a cooking spice in Europe and remains a popular ingredient in pickles, curry powders, and spice mixtures in India and Asia. In folk medicine, fenugreek has been used in the treatment of boils, cellulitis, and tuberculosis. Fenugreek seeds have been used as an oral insulin substitute, and seed extracts have been reported to lower blood glucose levels ***(www wikipedia.org).***

**Anti-diabetic properties of fenugreek:** Fenugreek seeds have a strong transforming effect on blood lipid levels. This could significantly lower the risk of atherosclerosis. Fenugreek's antidiabetic property is comparable to cinnamon in its effectiveness. It controls glucose metabolism and through this assists in the treatment of Type II diabetes. Insulin resistance is reduced by fenugreek herb and blood glucose homeostasis is controlled. This effect of fenugreek is quite similar to the working of the common antidiabetic drug i.e. glibenclamide. Only difference being that fenugreek is a completely natural medicine.

**Saponin rich herb:** Fenugreek belongs to the category of saponin rich herb. It contains steroidal saponins called furostanolic saponins. The principal furostanolic saponin in fenugreek is diosgenin. Diosgenin has been proven to have various effects on cholesterol metabolism, specifically in lowering LDL plasma cholesterol levels. Other furostanols in fenugreek include gitogenin, tigogenin, smilagenin, sarsasaponin, protodioscin etc. In the present invention the major saponin is 'protodioscin' and not 'diosgenin'.

**Herbal Anti-diabetic medicines existing in the prior art:** A number of anti-diabetic medicines e.g. powders and syrups having innovative natural ingredients for sugar control are available in the market, but none of them discloses the presence of furostanolic-saponin-rich fraction (protodioscin) of fenugreek seed as mentioned in the present invention. Some of the existing anti-diabetic formulations are briefly discussed in **TABLE 1** below:

**TABLE 1:**

| **S.No.** | **PRODUCT NAME** | **COMPANY NAME** | **INGREDIENTS** | **REMARKS** |
|---|---|---|---|---|
| **1.** | Diabecon | Himalaya Drug India | Eugenia jambolana, Tinospora cordifolia, Pterocarpus marsupium, Ficus Glomerulata, Momordica charantia, Ocimum sanctum and Gymnema sylvestre | Mixture of 7 herbs and not a well-defined extract. |
| **2.** | Insumin | Sydler remedies Pvt. Ltd. India | Gymnema sylvertere, Mamordica charantia, Curcuma longa | Mixture of 3 herbs and not a well-defined extract. |
| **3.** | Diabetic care capsules | Shriram Herbals, India. | Gurmarin, Monordica charantia. | Mixture of 3 herbs and not a well-defined extract. |
| **4.** | M.V.H. Diabex | Mahaved Health care, India | Gudmar (Gymnema Sylvestre), Methi Dana (Trigonella Foenumgraecum), Jamun (Eugenic Jambolana), Jira Safed (Cuminum Cyminum), Karela (Momordica Charantia), Kalongi (Nigella Sativa), Bilav (Aegle Marmelos), Til (Sesomum Indicum), Kali Musli (Curculigo Orchioides Garrtn). | Mixture of 9 herbs and not a well-defined extract. |
| **5.** | BalanZ (Anti Diabetic Capsules) | Prakruti Bio Pharma Pvt.Lmt. | Gymnema Sylvestra, Eugenia Jambolana, Trignonella Foenum Graecum, Momardica Charantia, and Neem Leaves. | Mixture of 5 herbs and not a well-defined extract. |
| **6.** | Herbal Diabetes Medicine | Mahaved Health care, India | Basant Kusumaker Ras Giloy (Tinospora Cordifolia) | Mixture of herbs and vegetables and not a well-defined extract. |
| | | | Sudh Shilajit (Asphaltum Punjabinum) Amla (Phyllanthus Emblica) | |
| | | | Gudmar (Gymnema Sylvestre) | |
| | | | Haldi Curcuma Longa) | |
| | | | Jamun (Eugenic Jambolana) | |
| | | | Karela (Momordica Charantia) | |
| | | | Neem Patra (Azadirachta Indica) | |
| | | | Bilav Patra (Angle Marmelos) | |
| | | | Tribang Bhasm Methi (Trigonella Foenumgraecu). | |
| **7.** | Full Spectrum Fenugreek | Planetary Herbals, Canada | Fenugreek seeds | Contains powdered fenugreek and and does not disclose a well-defined extract or presense of furostanolic saponin. |
| **8.** | Promilin Fenugreek Extract | Source Naturals, Canada | Fenugreek seeds extract | Undefined composition. Presence of furostanolic saponins not disclosed. |
| **9.** | Fenusterols | Sabinsa Corporation, USA | Fenugreek Seeds | Contains 50% furostanolic Saponin. i.e. diosgenin and not protodioscin |

### Patents relating to natural anti-diabetic compositions existing in prior art:

A number of patents in the prior art disclose herbal compositions effective against diabetes. However, none of them discloses a novel, anti-diabetic, chemically defined, furostanolic-saponin-rich (FSR) fraction (>70% including approximately 30% protodioscin as the major fraction) isolated from only a single source i.e. fenugreek seeds, as in the present invention.

WO2008118011 (A1): Discloses a composition for the treatment of diabetes mellitus comprising a part or extract from a mixture of herbs viz. *Curcuma longa, Gymneme sylvestre, Momordica charantia, Trigonella foenum graecum* and at least one *Terminalia species.*

DE4236409 (C1): Explains the use of an oral composition comprising a combination of 20-80 wt.% garlic and 20-80 wt.% ground fenugreek seeds, optimally together with carriers and additives, for treating type II diabetes mellitus. The above described patent discloses the use of a powdered mixture of garlic and fenugreek seeds in its composition and not a chemically defined extract as in the present invention. CN1726952 (A): Discloses an extract of fenugreek seeds effective in diabetes treatment but same has not been defined. There is no disclosure regarding protodioscin content.

CN1814167 (A): Discloses a health product for diabetes, prepared from Oyster Shell extract, Se-enriched yeast, Cr-enriched yeast, zinc aspartate, L- magnesium aspartate, linolenic acid powder, alpha- Thioctic Acid, Bitter Melon, tataricum Linn extract, Fructus lycii, Dwarf Lilyturf Tuber extract, Cortex Mori extract, Fructus Corni extract and Fenugreek. The same is a complex mixture containing non-herbal ingredients unlike the present invention which is totally herbal and not a mixture but an extract. CN1720982 (A): Discloses an anti-hyperlipidemic composition of fenugreek seed, which comprises (by weight ratio) fenugreek seeds extract 30-90%, mulberry leaf extract 5-40%, astragalus root extract 3-30%. The fenugreek seeds extract contains total fiber 1-80%, the mulberry leaf extract contains total flavones 0.1-50%, and the astragalus root extract is the aqueous extract or organic solvent of astragalus root.

Thus, none of the patents of the prior art disclose a chemically defined, anti-diabetic, furostanolic-saponin-rich fraction (>70% including approximately 30% protodioscin as the major fraction) from fenugreek seeds, as in the present invention.

### OBJECTS OF THE INVENTION:

1. To provide a novel anti-diabetic furostanolic-saponin-rich fraction (>70%) which includes approximately 30% protodioscin as one of the bioactive components.
2. To provide a novel anti-diabetic furostanolic-saponin-rich fraction extracted from fenugreek seeds which is chemically defined.
3. To provide a novel anti-diabetic furostanolic-saponin-rich fraction extracted from fenugreek seeds which is highly potent and can be easily and conveniently formulated into modern, pharmaceutically acceptable forms e.g. capsules, tablets, syrups etc.
4. To provide a novel anti-diabetic furostanolic-saponin-rich fraction extracted from fenugreek seeds which is natural, safe, biodegradable, has no documented mammalian toxicity and is easily available commercially.
5. To disclose a bioactive fraction extracted from fenugreek seeds which is highly effective in the treatment and management of Type-1 and Type-2 diabetes.

### SUMMARY OF THE INVENTION:

The present invention discloses a novel anti-diabetic composition extracted from fenugreek seeds. The same comprises a chemically defined, furostanolic-saponin-rich fraction (>70% including approximately 30% protodioscin as the major fraction).

### DETAILED DESCRIPTION OF THE INVENTION:

The present invention discloses a novel, anti-diabetic, chemically defined, furostanolic-saponin-rich (FSR) fraction (>70% including approximately 30% protodioscin as the major fraction) isolated from fenugreek seeds. Since it does not contain any harmful chemicals and preservatives it is very safe and eco-friendly and does not produce any adverse effects on the health of the patients.

**NOVELTY ASPECT OF THE INVENTION:** The invention is a product. Specifically it is a chemically defined, anti-diabetic product i.e. a furostanolic-saponin-rich fraction (>70% including approximately 30% protodioscin as the major fraction) extracted from fenugreek seeds. Such a fraction has not been disclosed in the prior art.

**INVENTIVE STEP:** The inventive step of the present invention lies in
- extraction of the fraction
- identification of the same as anti-diabetic agent
- enabling its use as anti-diabetic agent by proving non-toxicity and working out therapeutically effective dose

After considerable research efforts, the inventors were able to extract the present fraction comprising furostanolic saponins (>70% including approximately 30% protodioscin as the major fraction) which is 100% natural, highly potent and quite efficacious in treatment and management of diabetes (Type 1 and Type 2).

**INDUSTRIAL APPLICATION:** Fenugreek seeds which are required for extracting the fraction are easily available commercially. The product i.e. furostanolic saponin rich fraction from fenugreek seeds comprising >70% furostanolic saponins with approximately 30% protodioscin as one of the major fractions, can easily be prepared on a commercial scale and effectively used as an anti-diabetic agent. The same is in powdered form and fully water soluble. Accordingly, it can be formulated into any of the standard oral dosage forms e.g. tablets, capsules or the like and being water soluble can be also added to drinks or formulated in liquid form for appropriate use as anti-diabetic agent.

### EXTRACTION PROCESS FOR OBTAINING FUROSTANOLIC RICH SAPONIN FRACTION (>70% INCLUDING APPROXIMATELY 30% PROTODIOSCIN) FROM FENUGREEK SEEDS:

The process comprises the following main steps:
(1) **Primary extraction:** Powdered fenugreek seeds are subjected to primary extraction with hydrophilic polar solvents specifically lower aliphatic alcohols such as methanol, ethanol and butanol.
(2) **Ion-exchange chromatography and secondary extraction:** The primary extraction step is followed by ion-exchange chromatography using HP-20/PA-500 mixed bed resin and a secondary extraction step involving use of a novel composite extraction solvent comprising chlorohydrocarbon: alcohol (1: 1, v/v) to obtain the desired, high-purity (>70%), bioactive, furostanolic saponin rich (FSR) fraction. Preferably, the chlorohydrocarbon used is methylene dichloride and alcohol is methanol.
(3) **Yield optimization:** For yield optimization, the entire process is carried out between pH 6-8, optimally near neutral pH and temperature below 80 degree Celsius to avoid loss of structural integrity and bioactivity of the desired FUROSTANOLIC SAPONINS. Hence, entire extraction process is carried out below 80 degree Celsius, preferably between 40-65 degree Celsius. In case, extraction is carried out at temperatures lower than the specified range, extraction efficiency is found to be reduced and leads to lower yields, thus affecting commercial viability.

### IDENTIFICATION OF ANTI-DIABETIC ACTIVITY

Experimental studies were performed in animal models (rats) to determine anti-diabetic potential if any (Type 1 and Type 2) of the novel fraction isolated from fenugreek seeds (>70% furostanolic saponins with approximately 30% protodioscin as one of the major fractions). The results of treatments given in Type-1 animal models are given in **Table 2** below:

**TABLE 2: EFFECT OF CHRONIC ADMINISTRATION OF FUROSTANOLIC-SAPONIN-RICH (FSR) FENUGREEK SEED EXTRACT ON PLASMA GLUCOSE LEVEL (PGL) IN NORMAL AND TYPE-1 DIABETIC RATS**

| **Group** | **Treatment** | **PGL (mg/dl) at different intervals (days (d)) (% PGL Reduction)** | | |
|---|---|---|---|---|
| | | **0d** | **7d** | **14d** |
| **Normal control** | Veh. saline (2ml/kg, p.o.) | 97.50 ± 4.96 | 94.17± 4.17 | 99.54 ± 4.50 |
| **Normal Treated** | FSR (450 mg/kg, p.o) | 96.67 ± 3.33 | 76.67 ± 3.80* (18.6 %) | 84.72 ± 2.94* (15.0%) |
| **Diabetic Treated** | FSR (150mg/kg, p.o.) | 522.00 ± 27.82 | 410.00 ± 24.35 (18.1%) | 507.54 ± 23.90 (-0.7%) |
| **Diabetic Treated** | FSR (450mg/kg, p.o.) | 527.88 ± 26.36 | 405.00 ± 22.50 (19.1 %) | 345.49 ± 25.99* (31.5%) |
| **Diabetic Treated** | INS 1 U/kg, i.p. | 527.98 ± 50.31 | 218.33 ± 16.87 (56.3 %) | 260.65 ± 22.96 (48.2 %) |

The figures in parenthesis indicate the percent PGL reduction as compared to respective vehicle control group. Abbreviations are FSR- Furostanolic Saponin Rich fraction from fenugreek seeds (->70% including approximately 30% protodioscin as the major fraction); INS- Insulin. PGL- Plasma Glucose Level. The test and reference items were administered once daily chronically for 14 days either orally (p.o.) or intra peritoneally (i.p.).

The data given above shows that treatment with the furostanolic saponin rich (FSR) fraction of the present invention (450 mg/kg, p.o. for two weeks) in Type-1 diabetic rats showed significant plasma glucose lowering activity (31.5 %), comparable to that of insulin (48.2 % with 1 U/kg, i.p.). In addition, FSR at the same dose showed slight hypoglycemic activity (15%) in normal rats.

I In Type-2 diabetic rats, the isolated fraction alone and in combination with the anti-diabetic drug, metformin produced significant reduction of plasma glucose levels. The reduction in plasma glucose was more in combination with metformin, indicating synergistic effect.

### ENABLING USE OF THE FRACTION AS ANTI-DIABETIC AGENT

### Toxicity and Efficacy studies in animals (Pre-clinical studies)

The use of the fraction as an anti-diabetic agent was enabled by proving its non-toxicity and anti-diabetic effect in animal models and subsequently working out the therapeutically effective and safe dose in humans.

**Toxicity Studies in animal models:** Single dose acute toxicity studies (15 days) were carried out in Swiss albino mice as per International OECD GLP guidelines (1998) at dose levels of 500 mg, 1000 mg and 2000 mg per kg body weight. No mortality or treatment related observable toxic effects were observed. Subsequently, repeated dose toxicity studies (28 days) carried out at dose levels of 250, 500 and 1000 mg per kg/day as per OECD guidelines also did not reveal any toxic effect indicating that the highest tested dose of 1000 mg/kg body weight for 28 days of the fraction may be considered as safe for mammals.

### Efficacy studies (Anti-diabetic effect) in animal models:

Studies in animal models revealed that the fraction is effective in controlling plasma glucose levels in both Type-1 and Type- 2 diabetes. Treatment with the furostanolic saponin rich (FSR) fraction of the present invention (450 mg/kg, p.o. for two weeks) in Type-1 diabetic rats showed significant plasma glucose lowering activity (31.5 %), comparable to that of insulin (48.2 % with 1 U/kg, i.p.). In addition, FSR at the same dose showed slight hypoglycemic activity (15%) in normal rats.

In Type-2 diabetic rats, the isolated fraction alone and in combination with the anti-diabetic drug, metformin produced significant reduction of plasma glucose levels. The reduction in plasma glucose was more in combination with metformin, indicating synergistic effect.

**Clinical studies:** Keeping in view highly purified nature of the extract, various dosage forms of the same were evaluated. Eventually dosage form as 500 mg capsules was found to be the most suitable owing to ease of formulation and avoidance of excipients resulting in a 100% natural, well-defined dosage form. Anti-diabetic potential of the dosage form was evaluated as a dietary supplement in human volunteers (n = 10) and results of the study are discussed below:
The study findings were as follows:
   1. The mean (SD) age of patient was 55.92±9.72 years and the median duration of diabetes was 11 years. The average number of antidiabetic drugs received was 2 drugs (metformin and glipizide).
   2. Glycemic control (anti-diabetic activity) was assessed by checking the fasting plasma glucose (FPG) and post-prandial plasma glucose (PPG) before and after initiation of dietary supplement of the Fenugreek extract to the patient.
   3. The average dose of dietary supplement given to the patient was 500 mg once daily for the period of one month.
   4. The mean fasting plasma glucose before dietary supplement initiation was 148.67±31.44 mg/dl and the mean post- prandial plasma glucose was 194.40±34.86 mg/dl
   5. The mean fasting plasma glucose after dietary supplement initiation was 96.89±18.35 mg/dl and the mean post- prandial plasma glucose was 129.00±14.82 mg/dl
   6. The mean difference of FPG observed was 51.77±25.63 mg/dl. The fasting plasma glucose did differ significantly (p<0.001) after the Fenugreek treatment with the duration of exposure of 1 month
   7. The mean difference of PPG observed was 57.85±51.67 mg/dl. The post-prandial plasma glucose did differ significantly (p=0.025) after the Fenugreek treatment with the duration of exposure of 1 month

The above findings (from 10 patients) denote the significant anti-diabetic activity of the fraction of the present invention in terms of reduction of blood glucose levels. Additionally, it was found that the fraction did not interfere with the action of standard, synthetic anti-diabetic drugs such as metformin and glipizide. Rather it exerted synergistic effect, significantly enhancing their anti-diabetic potential.

## Claims

1. A fraction comprising furostanolic saponins isolated from fenugreek seeds wherein
- the fraction is anti-diabetic
- the major compound in the fraction is protodioscin, which represents approximately 30% of said fraction
- concentration of furostanolic saponins in the fraction is >70%

2. The fraction as claimed in claim 1 wherein concentration of protodioscin in the fraction is >30%.

3. The fraction as claimed in claim 1 wherein the same is in powdered form and fully water soluble.

## Patentansprüche

1. Fraktion, umfassend furostanolische Saponine, isoliert aus Bockshornkleesamen, wobei
- die Fraktion antidiabetisch ist
- es sich bei dem Hauptbestandteil der Fraktion um Protodioscin handelt, das ungefähr 30 % der Fraktion darstellt
- die Konzentration furostanolischer Saponine in der Fraktion > 70 % beträgt.

2. Fraktion nach Anspruch 1, wobei die Konzentration von Protodioscin in der Fraktion > 30 % beträgt.

3. Fraktion nach Anspruch 1, wobei diese in pulverisierter Form vorliegt und vollständig wasserlöslich ist.

## Revendications

1. Fraction comprenant des saponines furostanoliques isolée à partir de graines de fenugrec, dans laquelle
- la fraction est antidiabétique
- le composé principal de la fraction est la protodioscine, représentant environ 30% de ladite fraction
- la concentration en saponines furostanoliques dans la fraction est >70%

2. Fraction selon la revendication 1, dans laquelle la concentration en protodioscine dans la fraction est >30%.

3. Fraction selon la revendication 1, dans laquelle celle-ci est sous forme de poudre et entièrement soluble dans l'eau.
